# EUROPEAN PATENT APPLICATION

(11) **EP 4 502 159 A1**
(43) Date of publication of application: **05.02.2025**
(21) Application number: 23794826.0
(22) Date of filing: 15.03.2023
(51) Int. Cl.: C12N 15/113, C12N 15/85, C12N 15/90, C12N 5/10, C12N 9/22, A61K 35/17, A61P 35/00

(54) **ENGINEERED IMMUNE CELL WITH CIITA GENE KNOCK-OUT AND USE THEREOF**

(30) Priority: 28.04.2022 CN 202210460793
(71) Applicant: Nanjing Bioheng Biotech Co., Ltd, Nanjing, Jiangsu 210061 (CN)
(72) Inventor: ZHOU, Yali, Nanjing, Jiangsu 210061 (CN); GUO, Tingting, Nanjing, Jiangsu 210061 (CN); TANG, Yingxiu, Nanjing, Jiangsu 210061 (CN); ZHANG, Xuejian, Nanjing, Jiangsu 210061 (CN); REN, Jiangtao, Nanjing, Jiangsu 210061 (CN)
(74) Representative: Rimini, Rebecca
(86) International application number: PCT/CN2023/081624
(87) International publication number: WO 2023/207390

(57) **Abstract**

Disclosed herein are an engineered immune cell with CIITA gene knock-out and use thereof. According to the present invention, an sgRNA specifically targeting CIITA gene is designed and synthesized, which can accurately target CIITA gene to achieve gene knock-out with high knockout efficiency. The provided sgRNA can be used for preparing a universal CAR-T cell.

## Description

### Cross-reference to related applications

The present disclosure claims priority to the Chinese patent application with application number CN202210460793.X and title "ENGINEERED IMMUNE CELL WITH CIITA GENE KNOCK-OUT AND USE THEREOF" filed with the Chinese Patent Office on April 28, 2022, the entire contents of which are incorporated herein by reference.

### Technical Field

The present disclosure belongs to the field of biomedicine. More specifically, the present disclosure relates to an engineered immune cell with CIITA gene knock-out and use thereof.

### Background Art

In recent years, cancer immunotherapy technology has developed rapidly, especially chimeric antigen receptor T cell (CAR-T)-related immunotherapy has achieved excellent clinical effects in the treatment of hematological tumors. In CAR-T cell immunotherapy, T cells are genetically modified *in vitro* to enable them to recognize tumor antigens, and after expanding to a certain number, they are infused back into the patient's body to kill cancer cells, thereby achieving the purpose of treating tumors.

In 2017, two autologous CAR-T therapies were approved by the FDA for marketing in US, one for B-cell acute leukemia and the other for diffuse B-cell non-Hodgkin's lymphoma. Although these two CAR-T cells have excellent clinical therapeutic effects, they are very expensive and have a long preparation cycle, which makes large-scale promotion very difficult. Therefore, it is necessary to develop universal CAR-T products that can be used for allotransplantation (allogeneic transplantation) to solve the above problems. Universal CAR-T can be prepared by using T cells isolated from the peripheral blood of healthy donors, thereby achieving allogeneic transfusion, and greatly shortening the time patients have to wait for treatment. In addition, the vitality and function of T cells obtained from healthy donors are also superior to those of patient-derived T cells, which can increase the CAR infection rate and improve the therapeutic effect.

However, the development of universal CAR-T cells still faces the following two problems: (1) after the engineered CAR-T cells enter the patient's body and proliferate to a certain extent, they may attack the patient's normal cells or tissues, thereby causing graft-versus-host disease (GvHD); and (2) the normal immune system in the patient's body may reject allogeneic CAR-T cells, thereby causing host-versus-graft disease (HvGD). Currently, the main method to reduce the risk of transplantation is to knock out TCR, MHC class I molecules and/or class II molecules.

CIITA is a main control factor for the expression of MHC class II molecules. CIITA includes an acidic amino acid-rich N-terminal, a PST region rich in Pro, Ser, and Thr, a middle GTP binding region, and a C-terminus rich in Leu repetitive sequences (LRRs), in which the N-terminal acidic region and the PST region are transcriptional activation regions.

### Summary

The present disclosure aims to provide an sgRNA specifically targeting the CIITA gene and an engineered immune cell in which the CIITA gene is knocked out using the sgRNA, and its use in disease prevention and/or treatment and/or diagnosis and treatment of cancer, infection or autoimmune diseases.

In a first aspect, the present disclosure provides an sgRNA comprising a spacer sequence as set forth in any one of SEQ ID NOs: 1-6, 10, 11, 15, 16, 19, 21, and 23. Preferably, the spacer sequence is as set forth in any one of SEQ ID NOs: 4, 6, 11, 15, 21, and 23. More preferably, the spacer sequence is as set forth in any one of SEQ ID NOs: 4, 6, 11, and 15.

In a second aspect, the present disclosure provides a nucleic acid encoding the above sgRNA and a vector comprising the nucleic acid.

In a third aspect, the present disclosure provides a method for knocking out the CIITA gene *in vitro,* comprising introducing a Cas nuclease and the above sgRNA into the cell.

In an embodiment, the Cas nuclease is Cas9, Cpf1 or Cas13a, and is present in the form of a protein, an encoding nucleic acid thereof, or a vector. Preferably, the Cas nuclease is Cas9.

In an embodiment, the sgRNA is present in the form of a RNA, an encoding nucleic acid, or a vector thereof.

In an embodiment, the cell is an immune cell, and the immune cell is a T cell, a B cell, a macrophage, a dendritic cell, a monocyte, a NK cell, and/or a NKT cell and the like. Preferably, the immune cell is a T cell, a NK cell or a NKT cell. More preferably, the T cell is a CD4+CD8+ T cell, a CD4+ T cell, a CD8+ T cell, a memory T cell, a naive T cell, a γδ-T cell, and/or an αβ-T cell.

In an embodiment, the method further comprises introducing the nucleic acid encoding the chimeric antigen receptor or the T cell receptor or both into the immune cell.

In an embodiment, the chimeric antigen receptor comprises a ligand binding domain, a transmembrane domain, a co-stimulatory domain and a primary signaling domain.

In an embodiment, the ligand binding domain binds to the following targets: CD7, TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin, IL-1 IRa, PSCA, PRSS21, VEGFR2, LewisY, CD24, PDGFR-β, SSEA-4, CD20, Folate receptor α, ERBB2 (Her2/neu), MUC1, EGFR, NCAM, Claudin18.2, Prostase, PAP, ELF2M, Ephrin B2, IGF-I receptor, CAIX, LMP2, gploo, bcr-abl, tyrosinase, EphA2, Fucosyl GMI, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-la, MAGE-A1, legumin, HPV E6, E7, MAGE AI, ETV6-AML, sperm protein 17, XAGE1, Tie 2, MAD-CT-1, MAD-CT-2, Fos-related antigen 1, p53, p53 mutant, prostate-specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin BI, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGFβ, APRIL, NKG2D or any combination thereof. Preferably, the target is selected from the group consisting of: CD7, CD19, CD20, CD22, BAFF-R, CD33, EGFRvIII, BCMA, GPRC5D, PSMA, ROR1, FAP, ERBB2 (Her2/neu), MUC1, EGFR, CAIX, WT1, NY-ESO-1, CD79a, CD79b, GPC3, Claudin18.2, NKG2D or any combination thereof. Those skilled in the art can determine the antigen to be targeted based on the condition to be treated, thereby designing the corresponding chimeric antigen receptor.

In an embodiment, the ligand binding domain comprises a CD19 and/or CD22-targeting antibody or antigen binding fragment thereof. Preferably, the ligand binding domain of the present disclosure comprises a light chain variable region sequence having at least 90%, at least 95%, at least 97%, at least 99% or 100% sequence identity to the amino acid sequence as set forth in SEQ ID NO: 28 and a heavy chain variable region sequence having at least 90%, at least 95%, at least 97%, at least 99% or 100% sequence identity to the amino acid sequence as set forth in SEQ ID NO: 29.

In an embodiment, the transmembrane domain is a transmembrane domain of a protein selected from the group consisting of: a TCRα chain, a TCRβ chain, a TCRγ chain, a TCRδ chain, a CD3ζ subunit, a CD3ε subunit, a CD3γ subunit, a CD3δ subunit, CD45, CD4, CD5, CD8α, CD9, CD16, CD22, CD33, CD28, CD37, CD64, CD80, CD86, CD134, CD137, CD154 or any combination thereof. Preferably, the transmembrane domain is derived from a CD8α chain, which has at least 70%, preferably at least 80%, more preferably at least 90%, at least 95%, at least 97%, at least 99% or 100% sequence identity to the amino acid sequence as set forth in SEQ ID NO: 31, or the coding sequence of the CD8α transmembrane domain has at least 70%, preferably at least 80%, more preferably at least 90%, at least 95%, at least 97%, at least 99% or 100% sequence identity to the nucleotide sequence as set forth in SEQ ID NO: 32.

In an embodiment, the chimeric antigen receptor further comprises a hinge region located between the ligand binding domain and the transmembrane domain. Preferably, the hinge region comprises a hinge region of a CD8α chain, an FcγRIIIα receptor, IgG4 or IgG1, more preferably a CD8α hinge region, which has at least 70%, preferably at least 80%, more preferably at least 90%, at least 95%, at least 97%, at least 99% or 100% sequence identity to the amino acid sequence as set forth in SEQ ID NO: 33, or the coding sequence of the CD8α hinge has at least 70%, preferably at least 80%, more preferably at least 90%, at least 95%, at least 97%, at least 99% or 100% sequence identity to the nucleotide sequence as set forth in SEQ ID NO: 34.

In an embodiment, the primary signaling domain is a signaling domains of a protein selected from the group consisting of: FcRγ, FcRβ, CD3γ, CD3δ, CD3ε, CD3ζ, CD22, CD79a, CD79b, CD66d, or any combination thereof. Preferably, the primary signaling domain comprises a CD3ζ primary signaling domain, which has at least at least 70%, preferably at least 80%, more preferably at least 90%, at least 95%, at least 97%, at least 99% or 100% sequence identity to the amino acid sequence as set forth in SEQ ID NO: 35 or 37, or the coding sequence thereof has at least 70%, preferably at least 80%, more preferably at least 90%, at least 95%, at least 97%, at least 99% or 100% sequence identity to the nucleotide sequence as set forth in SEQ ID NO: 36 or 38.

In an embodiment, the co-stimulatory domain includes but is not limited to a co-stimulatory signaling domain derived from the following proteins: TLR1, TLR2, TLR3, TLR4, TLR5, TLR6, TLR7, TLR8, TLR9, TLR10, CARD11, CD2, CD7, CD8, CD18 (LFA-1), CD27, CD28, CD30, CD40, CD54 (ICAM), CD83, CD134 (OX40), CD137 (4-1BB), CD150 (SLAMF1), CD152 (CTLA4), CD223 (LAG3), CD270 (HVEM), CD272 (BTLA), CD273 (PD-L2), CD274 (PD-L1), CD276 (B7-H3), CD278 (ICOS), CD357 (GITR), DAP10, LAT, NKG2C, SLP76, PD1, LIGHT, TRIM, ZAP70 or any combination thereof. Preferably, the co-stimulatory domain is selected from 4-1BB, CD28 or 4-1BB+CD28, more preferably 4-1BB co-stimulatory domain. The 4-1BB co-stimulatory domain has at least 70%, preferably at least 80%, more preferably at least 90%, at least 95%, at least 97%, at least 99% or 100% sequence identity to the amino acid sequence as set forth in SEQ ID NO: 39, or the coding sequence of the co-stimulatory domain has at least 70%, preferably at least 80%, more preferably at least 90%, at least 95%, at least 97%, at least 99% or 100% sequence identity to the nucleotide sequence as set forth in SEQ ID NO: 40.

In an embodiment, the chimeric antigen receptor further comprises a signal peptide, which has at least 70%, preferably at least 80%, more preferably at least 90%, at least 95%, at least 97% or at least 99% or 100% sequence identity to the amino acid sequence as set forth in SEQ ID NO: 41, or the coding sequence of the signal peptide has at least 70%, preferably at least 80%, more preferably at least 90%, at least 95%, at least 97% or at least 99% or 100% sequence identity to the nucleic acid molecule as set forth in SEQ ID NO: 42.

In an embodiment, the T cell receptor targets one or more of HBV, HPV E6, NYESO, mNY-ESO, WT1, MART-1, MAGE-A3, MAGE-A4, P53, Thyroglobulin, Tyrosinase.

In an embodiment, the chimeric antigen receptor or T cell receptor can be introduced into a host cell via a vector. Specifically, the vector is selected from the group consisting of: plasmid, retrovirus, lentivirus, adenovirus, vaccinia virus, Rous sarcoma virus (RSV), polyoma virus and adeno-associated virus (AAV), phage, phagemid, cosmid or artificial chromosome. In some embodiments, the vector further comprises elements such as an origin of autonomous replication in a host cell, a selectable marker, a restriction enzyme cleavage site, a promoter, a poly-A tail (polyA), a 3' UTR, a 5' UTR, an enhancer, a terminator, an insulator, an operon, a selectable marker, a reporter gene, a targeting sequence, and/or a protein purification tag. In a specific embodiment, the vector is a plasmid, a lentiviral vector, an AAV vector, an adenoviral vector or a retroviral vector.

In the fourth aspect, the present disclosure provides an engineered immune cell produced by the above method of knocking out the CIITA gene.

In an embodiment, the engineered immune cell further comprises suppressed or silenced expression of at least one gene selected from the group consisting of: GR, dCK, TCR/CD3 genes (e.g., TRAC, TRBC, CD3γ, CD3δ, CD3ε, CD3ζ), MHC related genes (HLA-A, HLA-B, HLA-C, B2M, HLA-DPA, HLA-DQ, HLA-DRA, TAP1, TAP2, LMP2, LMP7, RFX5, RFXAP, RFXANK, CIITA) and immune checkpoint genes such as PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2 and GUCY1B3. Preferably, the engineered immune cell further comprises suppressed or silenced expression of at least one gene selected from the group consisting of: TRAC, TRBC, HLA-A, HLA-B, HLA-C, B2M, RFX5, RFXAP, RFXANK, CIITA, PD1, LAG3, TIM3, CTLA4, more preferably TRAC, TRBC, HLA-A, HLA-B, HLA-C, B2M, RFX5, RFXAP, RFXANK, CIITA. More preferably, CIITA and TRAC in the engineered immune cell are knocked out, and this inactivation makes the TCR-CD3 complex non-functional in the cell. This strategy is particularly useful for avoiding graft-versus-host disease (GvHD).

In a fifth aspect, the present disclosure further provides a composition comprising the above engineered immune cells, and the use of the immune cell or composition in the preparation of a medicament for treating/preventing/diagnosing cancer, infection or autoimmune disease.

In an embodiment, the composition further comprises one or more pharmaceutically acceptable excipients.

In an embodiment, the engineered immune cell or composition of the present disclosure may further be used in combination with one or more additional chemotherapeutic agents, biological agents, drugs or treatments. In this embodiment, the chemotherapeutic agent, biological agent, drug or treatment is selected from radiotherapy, surgery, antibody reagents, small molecules or any combination thereof.

The advantage of the present disclosure is that the screened sgRNA can efficiently knock out the CIITA gene, thereby reducing or avoiding the immune rejection reaction of CD4+T cells to the allogeneic CAR-T cells, and preparing universal CAR-T cells by simultaneously knocked out the TRAC gene.

The present disclosure will be described in detail below with reference to the accompanying drawings and examples. It should be noted that those skilled in the art should understand that the drawings and the embodiments of the present disclosure are only for the purpose of illustration, and shall not constitute any limitation to the present disclosure. In the case of no contradiction, the embodiments in the present application and the features in the embodiments can be combined with each other.

### Brief Description of Drawings

FIG. 1 shows the knock-out efficiency of TRAC/CIITA genes in TRAC/HLA-II double knock-out CD19 CAR-T cells (UCAR19).
FIG. 2 shows the expression level of CAR in TRAC/HLA-II double knock-out CD19 CAR-T cells (UCAR19).
FIG. 3 shows the secretion levels of IL2 and IFNγ in TRAC/HLA-II double knock-out CD19 CAR-T cells (UCAR19).

### Detailed Description of Embodiments

### Detailed Description of Embodiments

Unless otherwise defined, all technical and scientific terms used herein have the same meaning as commonly understood by ordinary technicians in the field to which this application belongs. In order to make it easier to understand this application, certain terms are defined below.

### sqRNA

The CRISPR/Cas system is a natural immune system of prokaryotes. After being invaded by a virus, some bacteria can store a small segment of the viral gene in a storage space called CRISPR in their own DNA. When the virus invades again, the bacteria can recognize the virus based on the stored segment and cut off the viral DNA to render it ineffective. There are type I, type II and type III of CRISPR systems, among which CRISPR/Cas9 is the most studied as type II. In addition to Cas9 nuclease, Cas12a (also known as Cpf1) and Cas13a (also known as C2c2) are also commonly used Cas nucleases. CRISPR/Cas9-mediated gene editing technology can be used to generate transgenic models, regulate transcription, and regulate epigenetics, etc.

CRISPR/Cas9 system is mainly composed of Cas9 protein and single-stranded guide RNA (sgRNA), in which the Cas9 protein has the function of cutting DNA double-strand and the sgRNA plays a guiding role. The principle of this system is that CRISPR RNA (crRNA) bind to tracrRNA (trans-activating RNA) through base pairing to form tracrRNA/crRNA complex, which guides the nuclease Cas9 protein to cut double-stranded DNA at the sequence target site paired with the spacer sequence in the crRNA.

As used herein, the term "sgRNA", also known as "single guide RNA", refers to an artificially engineered RNA that is produced by fusing crRNA and tracrRNA molecules into a "single guide RNA". The crRNA may comprise a nucleic acid targeting segment (e.g., a spacer sequence) of a guide nucleic acid and a stretch of nucleotides that may form one half of the duplex of the double strand of the Cas protein binding segment of the guide nucleic acid. The tracrRNA may comprise a stretch of nucleotides that forms the other half of the duplex of the double strand of the Cas protein binding segment of the sgRNA. The stretch of nucleotides of crRNA can be complementary and hybridized to the stretch of nucleotides of tracrRNA to form the duplex of the double strand of the Cas protein binding segment of the guide nucleic acid. When sgRNA binds to the Cas9 nuclease, it is able to recognize and cut the DNA target specific to the guide RNA. The part of the sgRNA responsible for complementary pairing with the target DNA is the spacer sequence contained therein. In an embodiment, the sgRNA comprises a spacer sequence as set forth in any one of SEQ ID NOs: 1-6, 10, 11, 15, 16, 19, 21, and 23.

As used herein, the "PAM" is short for protospacer adjacent motif (PAM), refers to a short nucleotide sequence adjacent to the (targeted) target sequence recognized by the sgRNA/Cas nuclease system. If the target DNA sequence is not adjacent to a suitable PAM sequence, the Cas nuclease may not successfully recognize the target DNA sequence. The sequence and length of the PAM herein may vary depending on the Cas protein or Cas protein complex used, and can be AGG, TGG, CGG or GGG, etc.

The design of sgRNA spacer sequences is generally based on the following principles:
(1) The length should generally be around 20 nt;
(2) Base composition: the seed sequence (the 1st to 12th bases close to the PAM region) should avoid ending with more than 4 Ts, and the GC% content is preferably 40%-60%;
(3) The matching number between the seed sequence and the off-target site should be as low as possible;
(4) It is necessary to check whether there are SNPs in the genomic sequence of the target binding site;
(5) For the analysis of the off-target effect of the whole gene, the maximum allowable number of mismatched bases at the off-target site should be considered.

Therefore, the design of the sgRNA spacer needs to consider many factors, such as length, GC content, binding position of the target gene, binding rate with non-target sites, whether it contains SNPs, secondary structure, etc. At present, sgRNA can be designed through various online tools. However, since the Cas enzyme can cut any target sequence adjacent to the PAM site, for a specific target gene, the editing efficiency of a large number of sgRNAs designed by online tools is not the same, or even varies greatly. For example, the editing efficiency of the PAM site NGG is usually higher than that of NGA or NAG. The design of sgRNA is directly related to the gene editing efficiency of the CRISPR system, and sgRNA targets with high specificity will bring higher gene editing efficiency and more positive clones, which can achieve twice the result with half the effort in later screening and identification. Therefore, screening sgRNA with high specificity is crucial for improving the editing efficiency of the CRISPR system. Usually after designing sgRNA, *in vitro* cell activity screening is required to select highly specific sgRNA for subsequent experiments.

### Nucleic acid, vector

As used herein, the term "nucleic acid" can be DNA or RNA.

As used herein, the term "vector" is a nucleic acid molecule used as a vehicle for transferring genetic material into a cell, in which the nucleic acid molecule can be replicated and/or expressed.

In an embodiment, the vectors of the present disclosure include, but are not limited to, linear nucleic acid (e.g., DNA or RNA), plasmid, virus (e.g., retrovirus, lentivirus, adenovirus, vaccinia virus, Rous sarcoma virus (RSV), polyoma virus, and adeno-associated virus (AAV), etc.), phage, phagemid, cosmid, and artificial chromosome (including BAC and YAC). The vector itself is usually a nucleotide sequence, and usually is a DNA sequence compriseing an insert (transgene) and a larger sequence as "backbone" of the vector.

### Chimeric Antigen Receptor

As used herein, the term "chimeric antigen receptor" (CAR) comprises a ligand binding domain, a transmembrane domain, a co-stimulatory domain, and a primary signaling domain. "Ligand binding domain" refers to any structure or functional variant thereof that can bind to a ligand, including an antibody or antigen binding fragment thereof. The choice of ligand binding domain depends on the cell surface marker on the target cell associated with the specific disease state to be recognized, such as a tumor-specific antigen or a tumor-associated antigen. In an embodiment, the ligand binding domain comprises a CD19-targeting antibody or antigen binding fragment thereof.

As used herein, the term "antibody" includes monoclonal antibodies (including whole antibodies), polyclonal antibodies, multivalent antibodies, multispecific antibodies (e.g., bispecific antibodies), and antibody fragments or synthetic polypeptides carrying one or more CDR sequences capable of exhibiting the desired biological activity.

Typically, whole antibodies comprise two heavy chains and two light chains disulfide-bonded together, each light chain being disulfide-bonded to a respective heavy chain, to form a "Y" configuration. Both the heavy chain and the light chain contain a variable region and a constant region. The heavy chain variable region and the light chain variable region each includes three CDRs, and the CDRs are separated by more conserved framework regions (FRs). The variable region is responsible for the recognition and binding of the antigen, while the constant region can mediate the binding of the antibody to the host tissue or factor.

As used herein, the term "antigen-binding fragment" comprises only a portion of an intact antibody, and typically comprises the antigen-binding site of the intact antibody and thus retains the ability to bind antigen. Examples of antigen-binding fragments in the present disclosure include, but are not limited to: Fab, Fab', F(ab')2, Fd fragment, Fd', Fv fragment, scFv, disulfide-linked Fv (sdFv), antibody heavy chain variable region (VH) or light chain variable region (VL), linear antibody, "diabody" with two antigen binding sites, single domain antibody, nanobody, a natural ligand for the antigen or a functional fragment thereof.

As used herein, the term "transmembrane domain" refers to a polypeptide structure that enables expression of a chimeric antigen receptor on the surface of an immune cell (e.g., a lymphocyte, a NK cell, or a NKT cell), and guides a cellular response of the immune cell against the target cell. The transmembrane domain can be natural or synthetic, and also can be derived from any membrane-bound protein or transmembrane protein. The transmembrane domain is capable of signaling when the chimeric antigen receptor binds to the target antigen. In an embodiment, the transmembrane domain is derived from a CD8 α chain.

As used herein, the term "hinge region" generally refers to any oligopeptide or polypeptide that functions to connect the transmembrane domain to the ligand binding domain. Specifically, the hinge region serves to provide greater flexibility and accessibility to the ligand binding domain. The hinge region may comprise up to 300 amino acids, preferably 10 to 100 amino acids and most preferably 25 to 50 amino acids. The hinge region can be completely or partially derived from a natural molecule, for example, completely or partially from the extracellular region of CD8, CD4 or CD28, or completely or partially from an antibody constant region. In an embodiment, the hinge region comprises a CD8α hinge region.

As used herein, the term "primary signaling domain" refers to a protein portion that transduces an effector function signal and guides a cell to perform a specified function. The primary signaling domain is responsible for the intracellular primary signal transmission after the ligand binding domain binds to the antigen, thereby leading to the activation of immune cells and immune responses. The primary signaling domain is the cytoplasmic sequence of the T cell receptor and co-receptors that function together to initiate primary signaling following antigen receptor binding, as well as any derivative or variant of these sequences and any synthetic sequence having the same or similar function. The primary signaling domain can contain a number of immunoreceptor tyrosine-based activation motifs (ITAMs). In an embodiment, the primary signaling domain comprises a CD3 zeta primary signaling domain.

The chimeric antigen receptors of the present invention comprise one or more co-stimulatory domains. The co-stimulatory domain can be an intracellular functional signaling domain from a co-stimulatory molecule, which comprises the entire intracellular portion of the co-stimulatory molecule, or a functional fragment thereof. A "co-stimulatory molecule" refers to a cognate binding partner that specifically binds to a co-stimulatory ligand on a T cell, thereby mediating a co-stimulatory response (e.g., proliferation) of the T cell. co-stimulatory molecules include, but are not limited to, MHC class 1 molecules, BTLA, and Toll ligand receptors. In an embodiment, the co-stimulatory domain is selected from 4-1BB, CD28 or 4-1BB+CD28, more preferably 4-1BB co-stimulatory domain.

The chimeric antigen receptor of the present disclosure may further comprise a signal peptide such that when it is expressed in a cell such as a T cell, the nascent protein is directed to the endoplasmic reticulum and subsequently to the cell surface. The core of the signal peptide may comprise a long hydrophobic amino acid segment, which has a tendency to form a single α-helix. Signal peptides that can be used in the present disclosure are well known to those skilled in the art, for example, signal peptides derived from CD8α, IgG1, GM-CSFRα, and the like.

### Engineered immune cells

As used herein, the term "immune cell" refers to any cell of the immune system that has one or more effector functions (e.g., cytotoxic cell killing activity, secretion of cytokines, induction of ADCC and/or CDC). For example, the immune cell can be a T cell, a macrophage, a dendritic cell, a monocyte, a NK cell, and/or a NKT cell. In an embodiment, the immune cell is derived from a stem cell, such as an adult stem cell, an embryonic stem cell, a cord blood stem cell, a progenitor cell, a bone marrow stem cell, an induced pluripotent stem cell, a totipotent stem cell, or a hematopoietic stem cell, and so on. Preferably, the immune cell is a T cell. The T cell can be any T cell, such as in vitro cultured T cell, for example, primary T cell, or T cell from in vitro cultured T cell line, e.g., Jurkat, SupT1, etc., or T cell obtained from a subject. Examples of subject include humans, dogs, cats, mice, rats, and transgenic species thereof. The T cell can be obtained from a variety of sources, including peripheral blood monocytes, bone marrow, lymph node tissue, umbilical blood, thymus tissue, tissue from sites of infection, ascites, pleural effusion, spleen tissue, and tumors. The T cell also can be concentrated or purified. The T cell can be at any stage of development including, but not limited to, a CD4⁺/CD8⁺ T cell, a CD4⁺ helper T cell (e.g., Th1 cell and Th2 cells), a CD8⁺ T cell (e.g., cytotoxic T cell), a tumor infiltrating cell, a memory T cell, a naive T cell, aγδ-T cell, an αβ-T cell. In a preferred embodiment, the immune cell is a human T cell. The T cell can be isolated from the blood of a subject using a variety of techniques known to those of skill in the art, such as Ficoll.

The nucleic acid sequence encoding the chimeric antigen receptor can be introduced into an immune cell using conventional methods known in the art (e.g., by transduction, transfection, transformation).

In an embodiment, in order to reduce the risk of graft-versus-host disease, the engineered immune cell further comprises suppressed or silenced expression of at least one gene selected from the group consisting of: TRAC, TRBC, HLA-A, HLA-B, HLA-C, B2M, RFX5, RFXAP, RFXANK, CIITA, PD1, LAG3, TIM3, CTLA4, more preferably TRAC, TRBC, HLA-A, HLA-B, HLA-C, B2M, RFX5, RFXAP, RFXANK, CIITA.

### Composition

The present disclosure also provides a composition comprising the engineered immune cell body described in the present disclosure. In an embodiment, the composition further comprises one or more pharmaceutically acceptable excipients.

As used herein, the term "pharmaceutically acceptable excipient" refers to a carrier and/or excipient that is pharmacologically and/or physiologically compatible (i.e., capable of triggering a desired therapeutic effect without causing any undesired local or systemic effects) with the subject and active ingredient.

The pharmaceutical composition according to the present disclosure is suitable for multiple routes of administration. Generally, the administration is parenterally accomplished. Parenteral delivery methods comprise topical, intraarterial, intramuscular, subcutaneous, intramedullary, intrathecal, intraventricular, intravenous, intraperitoneal, intrauterine, intravaginal, sublingual, or intranasal administration.

The pharmaceutical composition according to the present disclosure also can be prepared in various forms, such as solid, liquid, gaseous or lyophilized forms, particularly the pharmaceutical composition can be prepared in the form of ointment, cream, transdermal patch, gel, powder, tablet, solution, aerosol, granule, pill, suspension, emulsion, capsule, syrup, elixir, extract, tincture or liquid extract.

The composition according to the present disclosure can further be administered in combination with one or more other agents suitable for treating and/or preventing the disease to be treated. Preferred examples of agent suitable for the combination include known anti-cancer medicines such as cisplatin, maytansine derivatives, rachelmycin, calicheamicin, docetaxel, etoposide, gemcitabine, ifosfamide, irinotecan, melphalan, mitoxantrone, sorfimer sodiumphotofrin II, temozolomide, topotecan, trimetreate glucuronate, auristatin E, vincristine and doxorubicin; peptide cytotoxins, such as ricin, diphtheria toxin, pseudomonas exotoxin A, DNase and RNase; radionuclides such as iodine 131, rhenium 186, indium 111, iridium 90, bismuth 210, bismuth 213, actinides 225 and astatine 213; prodrugs such as antibody-directed enzyme prodrugs; immunostimulatory agents such as platelet factor 4, and melanoma growth stimulating protein; antibodies or fragments thereof, such as anti-CD3 antibodies or fragments thereof, complement activators, heterologous protein domains, homologous protein domains, viral/bacterial protein domains and viral/bacterial peptides. In addition, the pharmaceutical composition of the present disclosure also can be used in combination with one or more other treatment methods, such as chemotherapy and radiotherapy.

### Therapeutic/preventive/diagnostic use

The engineered immune cells and compositions of the present disclosure can be used as drugs for the treatment/prevention/diagnosis of cancer, infection or autoimmune diseases.

In an embodiment, cancers that can be treated with the immune cells or compositions of the present disclosure include, but are not limited to, acute lymphoblastic leukemia (ALL), chronic lymphocytic leukemia (CLL), acute myeloid leukemia (AML), breast cancer, lung cancer, colorectal cancer, gastric cancer, pancreatic cancer, ovarian cancer, metastatic adenocarcinoma, liver metastases, sarcoma, osteosarcoma, esophageal cancer, eye cancer, head and neck cancer, biliary tract cancer, bladder cancer, bone cancer, neuroblastoma, melanoma, mesothelioma, glioblastoma, glioma, malignant glioma, liver cancer, non-small cell lung cancer (NSCLC), gangliocytoma, brain cancer, kidney cancer, and prostate cancer. Infectious diseases that can be treated with the immune cells or compositions of the present disclosure include, but are not limited to, infections caused by viruses, bacteria, fungi, and parasites. Autoimmune diseases that can be treated with the immune cells or compositions of the present disclosureinclude, but are not limited to, type I diabetes, celiac disease, Graves' disease, inflammatory bowel disease, multiple sclerosis, psoriasis, rheumatoid arthritis, Addison's disease, Sjögren's syndrome, Hashimoto's thyroiditis, myasthenia gravis, vasculitis, pernicious anemia, and systemic lupus erythematosus.

### Example 1. Design of sgRNA and knock-out of CIITA gene

Suitable PAM sites on the CIITA gene were selected to design the spacer sequence. The spacer sequence targets the exon of the CIITA gene, which is the unique target sequence on the CIITA gene. The crRNA containing the spacer sequence was fused with the tracrRNA to obtain a sequence of about 100 bp, which was artificially synthesized to obtain the sgRNA product.

T cells were stimulated with DynaBeads CD3/CD28 CTSTM (Gibco, Cat. No. 40203D) and cultured at 37°C and 5% CO2 for 3 days. Then, 10 µg of Cas9 protein and 10 µg of sgRNA were electrotransfected into the activated T cells using a BTX Agile Pulse Max electroporator (Harvard Apparatus BTX) at 400V and 0.7ms to obtain T cells with CIITA knock-out. After electrotransfection, the T cells were immediately placed in 1 ml of pre-warmed culture medium and cultured at 37°C and 5% CO2 in the presence of IL-2 (300 IU/ml). Five days later, the expression of CD7 was detected by flow cytometry using APC anti-human HLA-DR, DP, DQ Antibody (Biolegend, Catalog No. 361714) to obtain the knock-out efficiency of the CIITA gene. The spacer sequences in sgRNA and their knock-out efficiencies are shown in Table 1.

**Table 1 Spacer sequences of different sgRNAs and their knock-out efficiencies.**

| No. | SEQ ID NO | Sequence | Knock-out Efficiency |
|---|---|---|---|
| A1 | 1 | GGGUGAUGAAGAGACCAGGG | 82.2% |
| A2 | 2 | GGACUCACUCCAUCACCCGG | 87.7% |
| A3 | 3 | GGACUCCAGAUGCUGCAGGG | 83.0% |
| A2 | 4 | GGUGAGUGAGAACAAGAUCG | 96.4% |
| A2 | 5 | GGGAUUCAGGCAGCUCAACG | 80.5% |
| A2 | 6 | GGAGGGAGGAAAAGGCCGUG | 92.6% |
| A2 | 7 | GGCUGCCUUGAGCGACACGG | 72.0% |
| A8 | 8 | GGGAGUUUGCGUAAGCAAAG | 47.8% |
| A9 | 9 | GGACAAGCUGUCGGAAACAG | 55.5% |
| A2 | 10 | GGCCUCGGAAGACACAGCUG | 86.9% |
| A2 | 11 | GGUCGCUCAAGGCAGCCCUG | 95.8% |
| A2 | 12 | GGGUGGCUGAUGGAGCGAAG | 60.6% |
| A2 | 13 | GGGACAGGUAGGACCCAGCA | 62.4% |
| A14 | 14 | GGGUUCUGGGACAGACUGCG | 68.6% |
| A15 | 15 | GGGAACAAGAUCGGGGACGA | 94.1% |
| A16 | 16 | GGUUUAGGUCCCGAACAGCA | 88.0% |
| A17 | 17 | GGAAGUGGUAGAGGCACAGG | 74.6% |
| A18 | 18 | GGAGAAGUGGUAGAGGCACA | 74.0% |
| A19 | 19 | GGGCUGCCUGAAUCUCCCUG | 82.4% |
| A20 | 20 | GGUCUGUGUCGGGUUCUGGG | 54.8% |

As can be seen from Table 1, spacer sequences of different sgRNAs have different knock-out efficiencies for the CIITA gene. Among the 20 sequences tested, SEQ ID NOs: 1-6, 10, 11, 15, 16, 19, 21, and 23 showed the knock-out efficiencies of more than 80%, and only SEQ ID NOs: 4, 6, 11, and 15 showed the knock-out efficiencies of more than 90%.

In the above table, A4, A11 and A15 were selected for sequence modification, and the specific sequence information is shown in Table 2 (the underlined sequence in the sgRNA is complementary to the target sequence). T cells were stimulated with DynaBeads CD3/CD28 CTSTM (Gibco, Cat. No. 40203D) and cultured at 37°C and 5% CO2 for 3 days. Then, half the amount of Cas9 protein and sgRNA used above, that is, 5 µg of Cas9 protein and 5 µg of sgRNA were electrotransfected into activated T cells using a BTX Agile Pulse Max electroporator (Harvard Apparatus BTX) at 400 V and 0.7 ms to obtain T cells with CIITA knock-out. Knock-out detection was performed as described above, and the results are shown in Table 2.

**Table 2. A4, A11, A15 with Spacer sequences after sequence modification and their knock-out efficiency**

| No. | SEQ ID NO | Sequence | Knock-out Efficiency |
|---|---|---|---|
| A4 | 4 | GGUGAGUGAGAACAAGAUCG | 59.1% |
| A4-1 | 21 | UGAGUGAGAACAAGAUCG | 55.9% |
| A4-2 | 22 | CUGAGUGAGAACAAGAUCGG | 31.1% |
| A11 | 11 | GGUCGCUCAAGGCAGCCCUG | 58.6% |
| A11-1 | 23 | GUCGCUCAAGGCAGCCCUG | 61.1% |
| A11-2 | 24 | GUCGCUCAAGGCAGCCCUGU | 30.8% |
| A15 | 15 | GGGAACAAGAUCGGGGACGA | 64.6% |
| A15-1 | 25 | GAACAAGAUCGGGGACGA | 34.4% |
| A15-2 | 26 | GAGAACAAGAUCGGGGACGA | 49.8% |

As can be seen from Table 2, after sequence modification of A4, A11 and A15, the spacer sequences of sgRNA obtained by different modification methods also showed different CIITA gene knock-out efficiencies. The inventors found that adding one or two additional Gs at the 5' end can significantly improve the knock-out efficiency compared with sgRNA that is completely complementary to the target sequence. Specifically, A4-1 (18 nt) and A4-2 (20 nt) are completely complementary to the target sequence, while A4 has two additional Gs at the 5' end of A4-1. Compared with A4-2 and A4-1, A4 has significantly improved knock-out efficiency. Similarly, the knock-out efficiency of A11 is comparable to that of A11-1, and both are significantly higher than A11-2; and the knock-out efficiency of A15 is significantly higher than A15-1 and A15-2. Based on the knock-out efficiency, A15 was selected for subsequent experiments.

### Example 2. Preparation of CAR-T cells with TRAC/HLA-II knock-out

### 1. Preparation of CD19 CAR T cells

The sequences coding the following proteins were synthesized and cloned sequentially into the pGEM-T Easy vector (Promega, Cat. No. A1360) in sequence: CD8α signal peptide (SEQ ID NO: 41), anti-CD19 scFv (SEQ ID NO: 30), CD8α hinge region (SEQ ID NO: 33), CD8α transmembrane region (SEQ ID NO: 31), 4-1BB co-stimulatory domain (SEQ ID NO: 39), CD3ζ primary signaling domain (SEQ ID NO: 35), and the correct insertion of the target sequence was confirmed by sequencing.

3 ml Opti-MEM (Gibco, Cat. No. 31985-070) was added to a sterile tube to dilute the above plasmid, and then packaging vector psPAX2 (Addgene, Cat. No. 12260) and envelope vector pMD2.G (Addgene, Cat. No. 12259) were added according to the ratio of plasmid : viral packaging vector : viral envelope vector = 4:2:1. Then, 120 µl X-treme GENE HP DNA transfection reagent (Roche, Cat. No. 06366236001) was added, mixed immediately, and incubated at room temperature for 15 min, and then the plasmid/vector/transfection reagent mixture was added dropwise to the culture flask containing 293T cells. Viruses were collected at 24 hours and 48 hours, pooled, and ultracentrifuged (25000g, 4°C, 2.5 hours) to obtain concentrated lentivirus.

T cells were activated with DynaBeads CD3/CD28 CTSTM (Gibco, Cat. No. 40203D), and were further cultured for 1 day at 37°C and 5% CO₂. Then, the concentrated lentivirus was added, and after 3 days of continuous culture, CD19-targeting CAR-T cells were obtained.

10 µg of Cas9 protein, 5 µg of CIITA sgRNA (the spacer sequence as set forth in SEQ ID NO: 15), and 5 µg of TRAC sgRNA (the spacer sequence as set forth SEQ ID NO: 27) were electrotransfected into activated CD19 CAR-T cells using a BTX Agile Pulse Max electroporator (Harvard Apparatus BTX) at 400 V and 0.7 ms to obtain TRAC/HLA-II double knock-out CD19 CAR-T cells (UCAR19). CD19 CAR-T cells without knock-out were used as a positive control (cCAR19). Wild-type T cells without CAR transfection were used as negative controls (NT).

### 2. Editing efficiency and CAR level detection

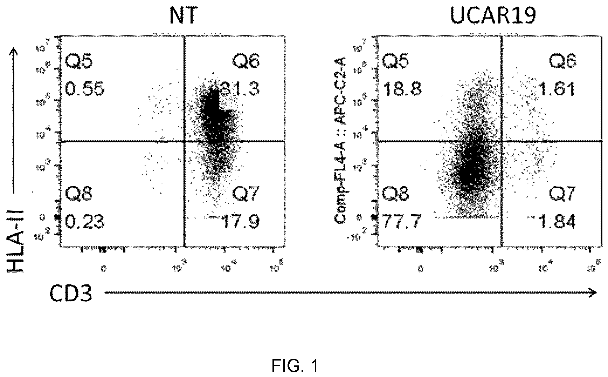

After culturing cCAR19 and UCAR19 cells at 37°C and 5% CO₂ for 11 days, the gene editing efficiency of TRAC and CIITA was detected using PE-anti human CD3 (BD Pharmingen, Catalog No. 300456) and APC anti-human HLA-DR, DP, DQ (Biolegend, Catalog No. 361714) antibodies by flow cytometry , and the results are shown in Figure 1.

It can be seen that TRAC and HLA-II in UCAR19 were effectively knocked out, indicating that the selected sgRNA of the present invention can effectively knock out the TRAC and CIITA genes.

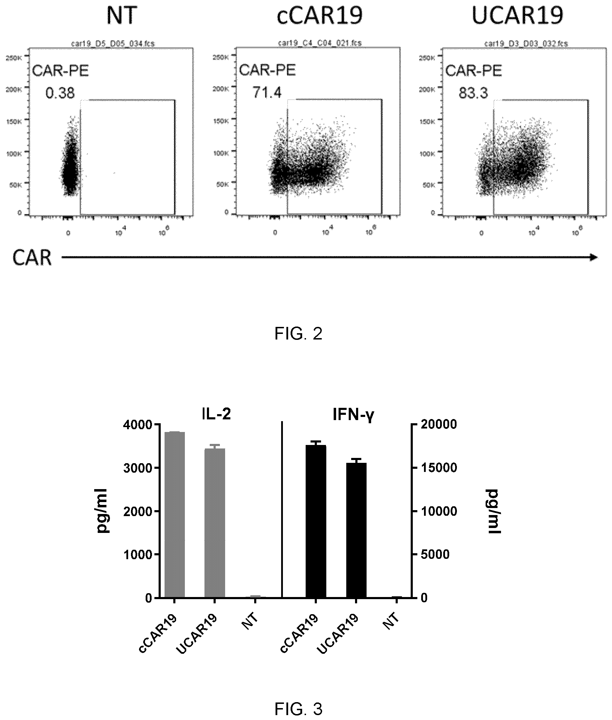

In addition, after culturing cCAR19 and UCAR19 cells at 37°C and 5% CO₂ for 11 days, the expression level of CAR on CAR T cells was detected using Biotin-SP (long spacer) AffiniPure Goat Anti-Mouse IgG, F(ab')₂ Fragment Specific(min X Hu, Bov, Hrs Sr Prot) (jackson immunoresearch, cat. no. 115-065-072) as the primary antibody and APC Streptavidin (BD Pharmingen, cat. no. 554067) or PE Streptavidin (BD Pharmingen, cat. no. 554061) as the secondary antibody by flow cytometry, and the results are shown in Figure 2.

It can be seen that the CAR expression level in cCAR19 is comparable to that in UCAR19, indicating that knock-out of the TRAC and CIITA genes does not affect the surface expression of CAR.

### Example 3. Cytokine secretion levels of CAR-T cells

When T cells kill target cells, the number of target cells decreases and cytokines such as IL2 and IFN-γ are released at the same time. According to the following steps, enzyme-linked immunosorbent assay (ELISA) was used to measure the release level of cytokine IL2 and IFNγ when CAR T cells killed target cells.

### (1) Collection of cell co-culture supernatant

Target cells (Raji) were plated in 96-well plates at 1×10⁵/well, and then the three groups of cells were co-cultured with target cells at a ratio of 1:1, and cell co-culture supernatants were collected after 18-24 hours.

### (2) Detection of the secretion of IL2 and IFN-γ in the supernatant by ELISA

The secretion of cytokine IL2 and IFNγ in the supernatant was measured by ELISA. A 96-well plate was coated with Purified anti-human IL-2 Antibody (Biolegend, Cat. No. 500302) and Purified anti-human IFN-γ Antibody (Biolegend, Cat. No. 506502) as capture antibody and incubated overnight at 4°C, and then the antibody solution was removed. 250 µL of PBST (1XPBS containing 0.1% Tween) solution containing 2% BSA (sigma, Cat. No. V900933-1kg) was added, and incubated at 37°C for 2 hours. Then, 50 µL of cell co-culture supernatant or standard was added to each well and incubated at 37°C for 1 hour. After washing the plate with 250 µL PBST (1XPBS containing 0.1% Tween), 50 µL of detection antibody Biotin anti-human IL-2 Antibody (Biolegend, Catalog No. 517605) and Anti-Interferon gamma antibody [MD-1] (Biotin) (abcam, Cat. No. ab25017) were added to each well and incubated at 37°C for 1 hour. Then HRP Streptavidin (Biolegend, Cat. No. 405210) was added, incubated at 37°C for 30 minutes, and the supernatant was discarded, washed with 250 µL PBST (1XPBS containing 0.1% Tween). 50 µL of TMB substrate solution was added to each well. Reactions were allowed to occur at room temperature in the dark for 30 minutes, after which 50 µL of 1 mol/L H₂SO₄ was added to each well to stop the reaction. Within 30 minutes after the reaction stopped, a microplate reader was used to detect the absorbance at 450 nm, and the content of cytokines was calculated according to the standard curve (plotted according to the reading value and concentration of the standard), the results are shown in Figure 3.

It can be seen that compared with the **NT** group, both cCAR19 cells and UCAR19 cells have a large amount of cytokine secretion. Moreover, when killing target cells, the secretion levels of IL2 and IFN-γ by cCAR19 cells and UCAR19 cells are comparable. This indicates that genes knock-out of TRAC and CIITA do not affect the killing function of CAR-T cells.

It should be noted that the above-mentioned are merely for preferred examples of the present disclosure and not used to limit the present disclosure. For one skilled in the art, various modifications and changes can be made to the present disclosure. Those skilled in the art should understand that any amendments, equivalent replacements, improvements, and so on, made within the spirit and principle of the present disclosure, should be covered within the scope of protection of the present disclosure.

## Claims

1. An sgRNA comprising a spacer sequence as set forth in any one of SEQ ID NOs: 1-6, 10, 11, 15, 16, 19, 21, and 23.

2. A nucleic acid encoding the sgRNA of claim 1.

3. A vector comprising the nucleic acid of claim 2.

4. A method for knocking out the CIITA gene *in vitro,* comprising introducing a Cas nuclease and an sgRNA into a cell, **characterized in that** the sgRNA is in the form of the RNA of claim 1, the nucleic acid of claim 2, or the vector of claim 3.

5. The method of claim 4, **characterized in that** the Cas nuclease is Cas9, Cas12a, or Cas13a.

6. The method of claim 4, **characterized in that** the cell is an immune cell, and the immune cell is a T cell, a B cell, a macrophage, a dendritic cell, a monocyte, a NK cell, or a NKT cell.

7. The method of claim 6, **characterized in that** the cell is a CD4⁺CD8⁺ T cell, a CD4⁺ T cell, a CD8⁺ T cell, a memory T cell, a naive T cell, a γδ-T cell, or an αβ-T cell.

8. The method of claim 6, **characterized in that** the immune cell is introduced with a nucleic acid encoding a chimeric antigen receptor and/or a T cell receptor.

9. The method of claim 8, **characterized in that** the chimeric antigen receptor comprises a ligand binding domain, a transmembrane domain, a co-stimulatory domain and a primary signaling domain, wherein the ligand binding domain targets one or more of CD7, CD19, CD20, CD22, BAFF-R, CD33, EGFRvIII, BCMA, GPRC5D, PSMA, ROR1, FAP, ERBB2, MUC1, EGFR, CAIX, WT1, NY-ESO-1, CD79a, CD79b, GPC3, Claudin18.2, and NKG2D.

10. The method of claim 9, **characterized in that** the ligand binding domain comprises a CD19 and/or CD22-targeting antibody or antigen binding fragment thereof.

11. The method of claim 10, **characterized in that** the CD19-targeting antibody or antigen-binding fragment thereof comprises a light chain variable region sequence having at least 90%, at least 95%, at least 97%, at least 99% or 100% sequence identity to the amino acid sequence as set forth in SEQ ID NO: 28 and a heavy chain variable region sequence having at least 90%, at least 95%, at least 97%, at least 99% or 100% sequence identity to the amino acid sequence as set forth in SEQ ID NO: 29.

12. The method of claim 9, **characterized in that** the transmembrane domain is a transmembrane domain of a protein selected from the group consisting of: a TCRα chain, a TCRβ chain, a TCRγ chain, a TCRδ chain, a CD3ζ subunit, a CD3ε subunit, a CD3γ subunit, a CD3δ subunit, CD45, CD4, CD5, CD8α, CD9, CD16, CD22, CD33, CD28, CD37, CD64, CD80, CD86, CD134, CD137, CD154 or any combination thereof.

13. The method of claim 9, **characterized in that** the co-stimulatory domain is a co-stimulatory signaling domain of a protein selected from the group consisting of: TLR1, TLR2, TLR3, TLR4, TLR5, TLR6, TLR7, TLR8, TLR9, TLR10, CARD11, CD2, CD7, CD8, CD18, CD27, CD28, CD30, CD40, CD54, CD83, CD134, CD137, CD150, CD152, CD223, CD270, CD272, CD273, CD274, CD276, CD278, CD357, DAP10, LAT, NKG2C, SLP76, PD1, LIGHT, TRIM, ZAP70 or any combination thereof.

14. The method of claim 9, **characterized in that** the primary signaling domain is a signaling domains of a protein selected from the group consisting of: FcRγ, FcRβ, CD3γ, CD3δ, CD3ε, CD3ζ, CD22, CD79a, CD79b, CD66d or any combination thereof.

15. An engineered immune cell obtained by the method of any one of claims 6-14, **characterized in that** the CIITA gene is knocked out.

16. The engineered immune cell of claim 15, **characterized in that** the engineered immune cell further comprises suppressed or silenced expression of at least one gene selected from the group consisting of: TRAC, TRBC, HLA-A, HLA-B, HLA-C, B2M, RFX5, RFXAP, RFXANK, CIITA, PD1, LAG3, TIM3, CTLA4 or any combination thereof.

17. The engineered immune cell of claim 16, **characterized in that** CIITA and TRAC in the engineered immune cell are knocked out.

18. A composition comprising the engineered immune cell of any one of claims 15-17.

19. Use of the engineered immune cell of any one of claims 15-17 or the composition of claim 18 in the preparation of a medicament for treating/preventing/diagnosing cancer, infection or autoimmune disease.
